# EUROPEAN PATENT SPECIFICATION

(11) **EP 1 461 340 B1**
(45) Date of publication and mention of the grant of the patent: **08.04.2009**
(21) Application number: 02785623.6
(22) Date of filing: 06.12.2002
(51) Int. Cl.: C07D 493/20, A61K 31/357, A61P 33/06, A61P 35/00

(54) **TRIOXANE DERIVATIVES AS ANTIMALARIA OR ANTICANCER COMPOUNDS**
TRIOXANDERIVATE ZUR VERWENDUNG ALS ANTIMALARIA- ODER ANTIKREBS-VERBINDUNGEN
DERIVES DE TRIOXANE COMME ANTIPALUDEENS OU ANTICARCINOGENES

(30) Priority: 06.12.2001 GB 0129214; 31.07.2002 GB 0217724
(43) Date of publication of application: 29.09.2004
(73) Proprietor: UFC LIMITED, Manchester M15 6SY (GB)
(72) Inventor: O'NEILL, Paul Michael, Liverpool L15 7JE (GB); HIGSON, Adrian Peter, Bury, Lancashire BL9 7JG (GB); TAYLOR, Sara, New Mills, Derbyshire SK22 3DD (GB); IRVING, Edward, Winsford, Cheshire CW7 2LE (GB)
(74) Representative: Wilson Gunn
(86) International application number: PCT/GB2002/005530
(87) International publication number: WO 2003/048168

(56) References cited:
- WO-A-99/33461

## Description

The present invention relates to certain compounds containing a trioxane moiety that have potent antimalarial activity and antitumour activity.

Artemisinin (1), which is also known as qinghaosu, is a tetracyclic 1,2,4-trioxane occurring in *Artemisia annua*. Artemisinin and its derivatives dihydroartemisinin (DHA) (2), artemether (3) and sodium artesunate (4) are used routinely in the treatment of malaria and have been found to be particularly effective against cerebral malaria.

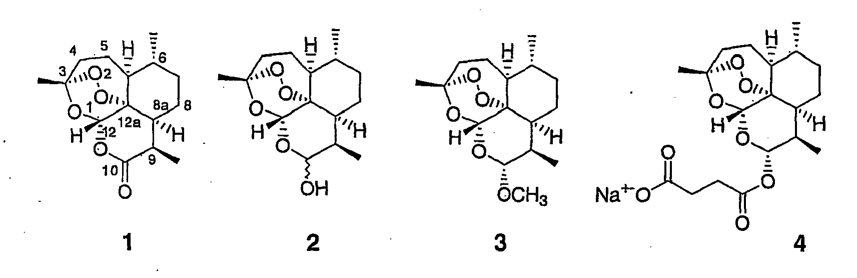

Different mechanisms of action have been proposed to account for the antimalarial action of artemisinin and its derivatives (Posner et al., J. Am. Chem. Soc. 1996, 118, 3537; Posner et al., J. Am. Chem. Soc. 1995, 117, 5885; Posner et al., J. Med. Chem. 1995, 38, 2273). Whilst the mechanism of action of artemisinin as an antimalarial has not been unequivocally established, it has been shown that the peroxide linkage is essential for antimalarial activity.

Certain artemisinin derivatives containing a peroxide moiety have also been tested for biological activity other than antimalarial activity. For example, the cytoxicity to Ehrlich ascites tumour cells of artemisinin, dihydroartemisinin, artemisitene, arteether, ethylperoxyartemisitene and an ether dimer of artemisinin has been demonstrated (Beekman et aL, Phytother. Res., 1996, 10, 140; Woerdenberg et al., J. Nat. Prod., 1993, 56, 849).

Alkylating agents remain an effective class of anticancer drugs, whose cytotoxic and therapeutic effects derive primarily from their ability to form intra- and inter-strand DNA crosslinks. Some dimeric chemical structures capable of cross-linking have especially high biological activities. These include bismustard lexitropsins (Chen et al., Bio. Med Chem. Lett., 1995, 5, 2223), dimeric cephalostatin analogues (Drögemüller et al., Eur. J. Org. Chem., 1998, 2811), bis-enediynes (Borman, Chem Eng. News, 1995, 28) and dimers of the saponin antitumour agent OSW-1 (Ma et al., Bio. Med Chem. Lett., 2001, 11, 2153).

Recently, there have been several reports of the potent antimalarial and antitumour activities of a series of synthetic dimers (5) (Posner, Bio. Med Chem., 1997, 5, 1257) and semisynthetic dimers (6) (Posner, J. Med. Chem., 1999, 42, 4275).

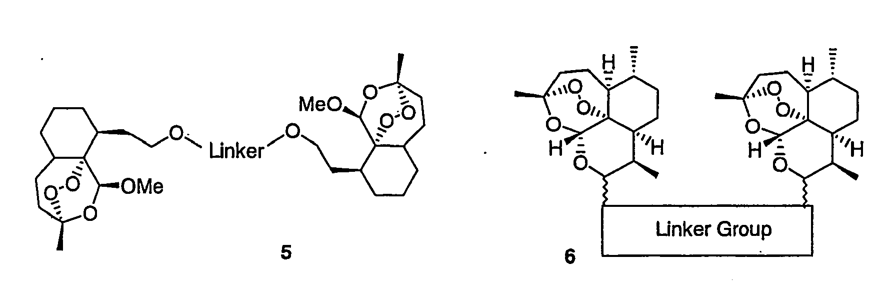

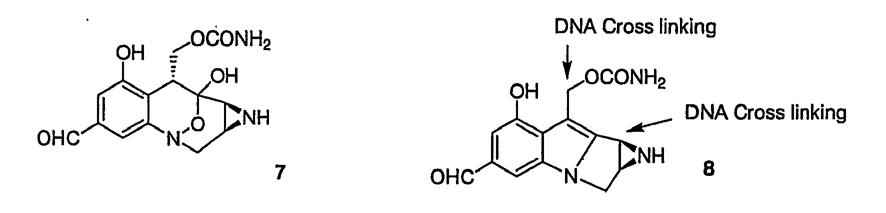

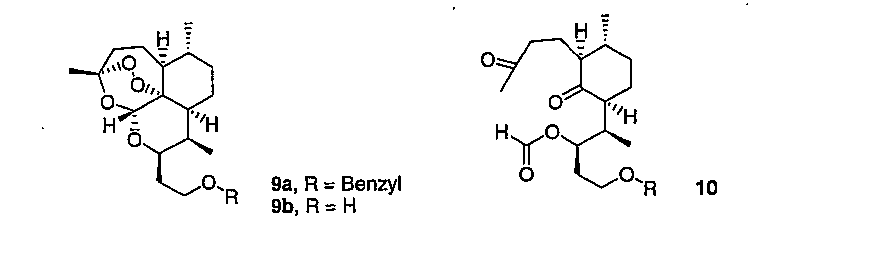

The linking groups in compound 6 as shown above were aromatic, benzoyl methylene or acetylenyl. The mechanism of antitumour and antimalarial activity of these trioxane dimers may involve either DNA-directed alkylation or protein alkylation, post reductive activation. Other antitumour agents, such as FR-900482 (7), upon reductive N-O scission, rearrange to produce cytotoxic mitosene like intermediates, (8), which can efficiently cross-link DNA. In a similar manner, we have recently shown that C-10 carba trioxane analogues of dihydroartemisinin (9a), upon reductive activation, generate potentially toxic dicarbonyl products (10) (O' Neill et al., J. Med. Chem., 1999). It was suggested that selective intraparasitic generation of compound 10 and subsequent protein alkylation may have a key role to play in the mechanism of action of these analogues.

Document WO 99/33461 discloses two step procedures for the replacement of the pyranose anomeric 10-OH group in dihydroartenisinin by a variety of carbon nucleophiles, resulting in C-10 carbon- substituted trioxane structures.

The present invention relates to the preparation of a series of dimers of a trioxane alcohol compound (9b).

According to a first aspect of the present invention therefore there is provided a compound of the following general formula: 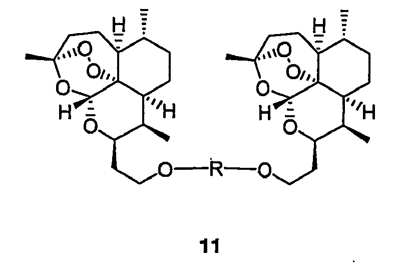 or a pharmaceutically acceptable salt thereof,
in which R is selected from the group comprising optionally substituted alkyl, aryl, bisalkyl or bisaryl ester, bisalkyl or bisaryl ether, acetal, ketal, boronate, silyl ether, carbamate, carbonate, sulphate, sulphonate, phosphate and phosphonate.

It will be appreciated that the preferred stereochmistry at the C-10 position is beta.

Since the compounds of the invention contain a carbon atom at the C-10 position, they are considerably more stable than first generation analogues, such as artemether, which contain the hydrolytically and chemically unstable C-10 acetal linkage. In addition, certain compounds of the invention have been shown to possess significantly improved antimalarial activity *in vitro* against *P. falciparum* malaria as compared to monomeric analogues in this class.

With regard to the optionally substituted alkyl and aryl groups, these are preferably of 1 to 20 carbon atoms, and are most preferably of 1 to 12 carbon atoms. The term "aryl" as used herein is intended to mean a compound or substituent having at least one aromatic ring and is particularly intended to include phenyl, biphenyl and heterocyclic aromatic rings of 5-6 atoms or bicyclic rings of up to 10 atoms. The term "alkyl" as used herein is intended to mean a saturated aliphatic hydrocarbon group, particularly a straight or branched carbon atom of 1-12 carbon atoms. The optionally substituted alkyl and aryl groups may be substituted by groups including, but not limited to, primary, secondary and tertiary amines; halogen-containing groups, such as bromide, chloride and fluoride; alcohols and derivatives thereof, including ethers and esters; and carboxylic acids and derivatives thereof, including esters and amides.

In one preferred embodiment of the invention, R represents any of the following groups:

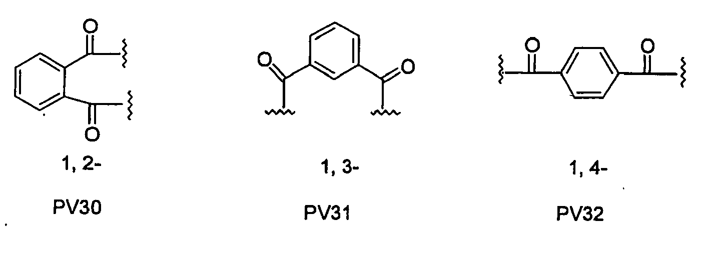

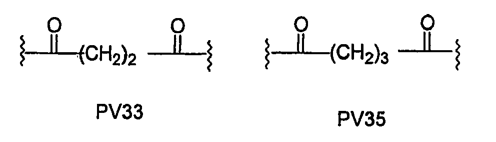

In another preferred embodiment of the invention, R represents any of the following groups:

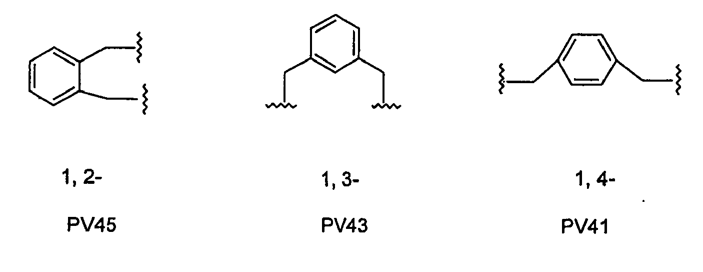

In a further preferred embodiment of the present invention, R represents a phosphate linker. Preferred phosphate linker groups include the following:

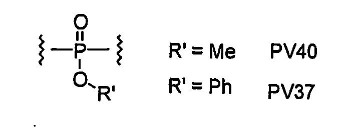

In further compounds exemplifying the present invention, R comprises any of the following groups:

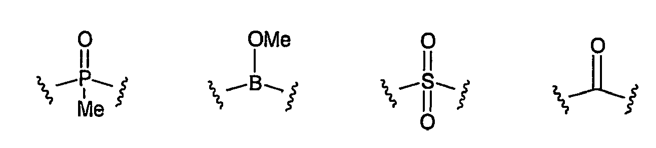

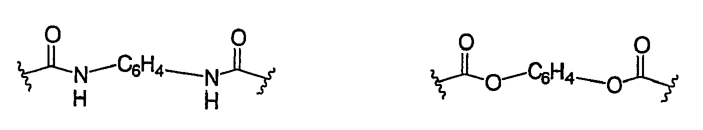

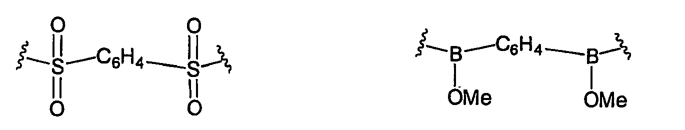

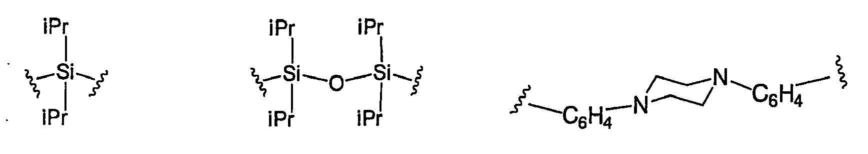

Those salts comprising pharmaceutically acceptable salts as referred to herein will be readily apparent to a skilled person. These salts include, but are not limited to acetate, adipate, besylate, bromide, camsylate, chloride, citrate, edisylate, estolate, fumarate, gluceptate, gluconate, glucuronate, hippurate, hyclate, hydrobromide, hydrochloride, iodide, isethionate, lactate, lactobionate, maleate, mesylate, methylbromide, methylsulfate, napsylate, nitrate, oleate, pamoate, phosphate, polygalacturonate, stearate, succinate, sulfate, sulfosalicylate, tannate, tartrate, terephthalate, tosylate, triethiodide, benzathine, calcium, diolamine, meglumine, olamine, potassium, procaine, sodium, tromethamine, and zinc.

A key intermediate for the preparation of a range of carba analogues of artemisinin, including parasite targeted amine analogues, water soluble sugar analogues (Jung and Bae, Heterocycles, 2000, 53, 261-262) and fluorinated benzyl ethers (O'Neill et al., J. Med. Chem., 1999, 42, 5487) is C-10-allyldeoxoartemisinin (12a).

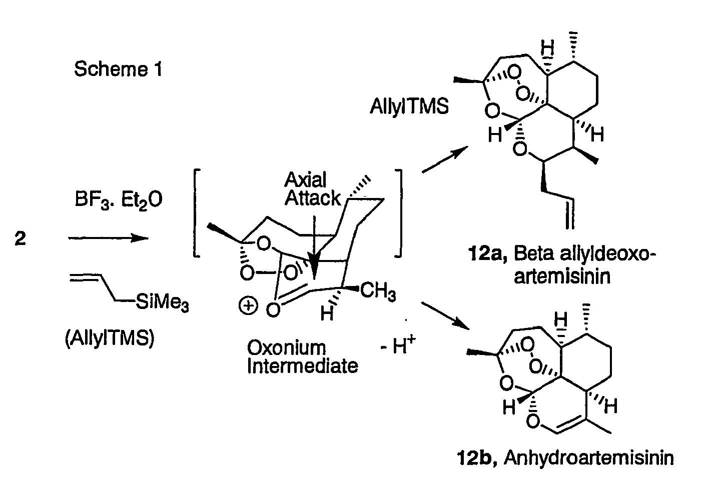

The originally reported method of synthesis of compound 12a involves the Lewis acid-mediated (BF₃.Et₂O) reaction of DHA with allyl trimethylsilane as carbon nucleophile (Pu and Ziffer et al., J. Med Chem., 1995, 38, 613). In this reaction, it is proposed that the product is derived by axial attack of allyltrimethylsilane on the intermediate oxonium ion (Scheme 1). A drawback of this procedure is the harsh nature of the Lewis acid, which results in the generation of substantial amounts of the dehydration product anhydroartemisinin (AHA) (12b). This makes purification of the desired material extremely difficult on a large scale since compounds 12a and 12b have very similar Rfs on silica gel.

We reasoned that a milder Lewis acid and a better anomeric leaving group would improve the yield of compound 12a. In considering the choice of Lewis acid, we also noted that hard Lewis acids such as BF₃.Et₂O, have the capacity to rearrange target C-10 carba derivatives whereas weaker Lewis acids such as zinc chloride do not. Indeed, in several large-scale runs using BF₃.Et₂O as Lewis acid, several unwanted products were observed including several deoxygenated trioxane by-products. With these observations in mind, zinc chloride was chosen as a preferred mild Lewis acid for the coupling reaction with C-10α-benzoate (13).

The benzoate (13) was prepared in high yield by treatment of DHA with benzoyl chloride in pyridine. NMR studies on the white crystalline solid indicated (α) stereochemistry with respect to the C-O linkage at C-10 (C-10 H appears as a doublet with a J₉₋₁₀ value of 9.9Hz indicating a dihedral angle of about 180° between itself and C-9 H). By way of example, reaction of the benzoate with allyltrimethylsilane (5 equivalents) in the presence of ZnCl₂ catalyst (1.2 equivalents) at 0°C for 4h gave the desired product, (12a), in 90% yield (Scheme 2). This result suggests that the leaving group and Lewis acid are perfectly tuned for effecting controlled oxonium generation and subsequent reaction with allylsilanes as nucleophiles. Ozonolysis of compound 12a and *in situ* reduction of the ozonide provides the key alcohol required for dimerisation, 10β-(2-hydroxyethyl)-deoxoartemisinin (9b).

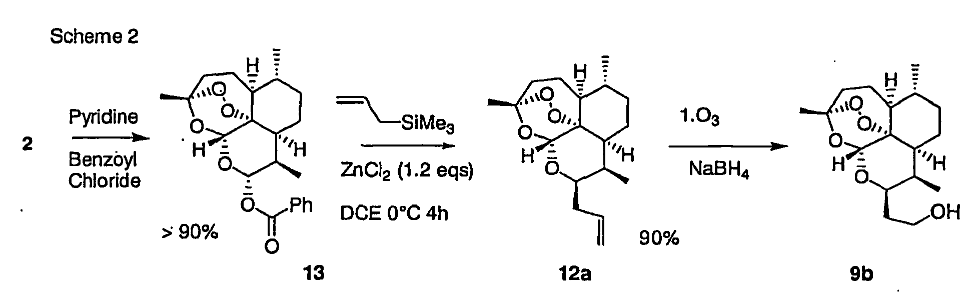

Thus, and in accordance with a second aspect of the present invention there is provided a process for the production of a compound according to claim 1 comprising reaction of the C-10 benzoate of dihydroartemisinin with allyltrimethylsilane in the presence of a weak Lewis acid to form 10β-allyldeoxoartemisinin (12a), and ozonolysis of the 10β-allyldeoxoartemisinin and subsequent reduction of the ozonide, to form 10β-(2-hydroxyethyl)-deoxoartemisinin (9b).

Preferably, zinc chloride is used as the weak Lewis acid.

Preferably also, crystalline C-10-α-benzoate is reacted with allyltrimethylsilane in the presence of zinc chloride in anhydrous dichloroethane as a solvent. Preferably also, the reaction is carried out in the presence of a dehydrating agent, such as 4Å molecular sieves. Furthermore, the reaction is preferably carried out under an inert atmosphere, such as an atmosphere of nitrogen. This reaction is preferably carried out at a temperature within the range of between -10°C to 0°C, and the preferred reaction time is around 4 hours.

In a particularly preferred embodiment of the present invention, 1.2 equivalents of zinc chloride is pre-mixed with 4.8 equivalents of allyltrimethylsilane in dichloroethane solvent (ca 5ml) at 0°C. The crystalline benzoate is then added at 0°C and the reaction allowed to stir for 4 hours.

The ozonide is preferably reduced *in situ*.

Trioxane dimers PV30-PV34 may be prepared from alcohol 9b and the requisite acid chloride and a catalytic quantity of 4-(dimethylamino) pyridine. For example, such dimers may be prepared by treating of alcohol 9b, preferably two equivalents of vacuum-dried alcohol 9b, and the requisite acid chloride, preferably one equivalent, and a catalytic quantity of 4-(dimethylamino) pyridine, as shown in Scheme 3. The reaction may be stirred overnight at room temperature and the solvent may be removed under reduced pressure. Products may be purified by column chromatography.

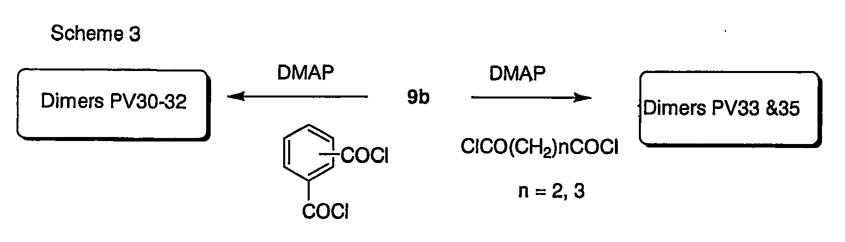

Trioxane ethers (PV45, PV43 and PV41) may be prepared using a newly developed and mild bis-reductive etherification of the O-trimethylsilyl ether derivative 14, which may be prepared in quantitative yield from 9b (Scheme 4). The reaction may be carried out using a bis-aldehyde, TMSOTf as a Lewis acid, preferably 0.2 equivalents, and triethylsilane as a reducing agent. The reaction temperature may be controlled during the reaction at temperatures from -78°C to-30°C. Using this process, the trioxane dimers have been obtained in yields of up to 74%.

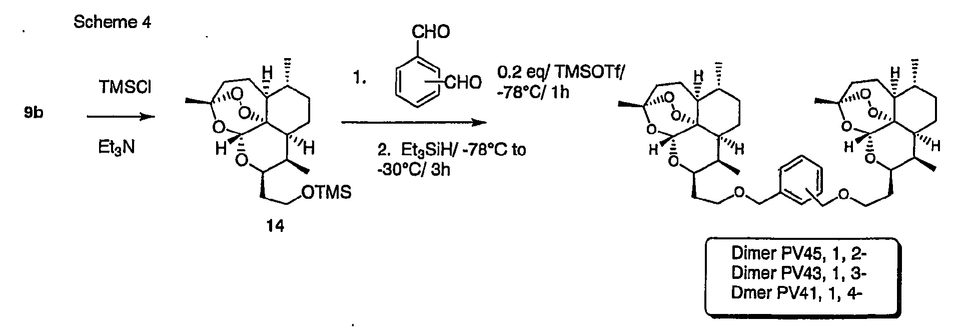

The phosphate esters of trioxane alcohol 9b may be prepared by treatment of the alcohol with sodium hexamethyldisilazide (NHMDS) followed by the appropriate dichlorophosphate as shown in Scheme 5. 10β-(2-hydroxyethyl) deoxoartemisinin, preferably two equivalents, may be reacted with dichlorophosphate, preferably one equivalent, in the presence of NHMDS, preferably one equivalent, as a base, the preferred solvent being anhydrous THF.

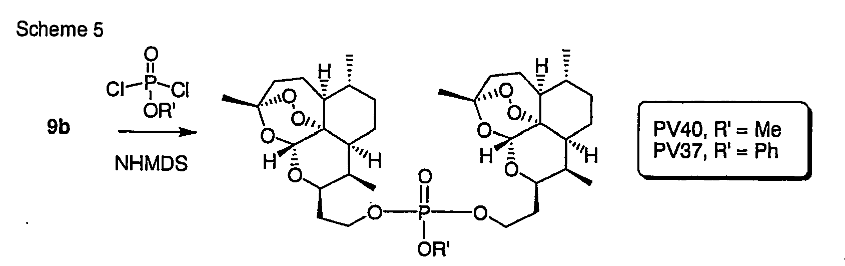

The new synthetic routes of the present invention provide the dimers in high chemical yield as single diastereoisomers.

The antimalarial activity of the new dimers was tested against the chloroquine-resistant K1 strain of *P. falciparum.* A single uncloned K1, chloroquine resistant strain of *P. falciparum* from Thailand was used. Parasites were maintained in continuous culture using the method of Trager and Jenson. (J. Parasitol., 1977, 63, 883-886). Cultures were grown in flasks containing human erythrocytes (2-5%) with parasitemia in the range of 1% to 10% suspended in RPMI 1640 medium supplemented with 25 mM HEPES and 32 mM NaHCO₃, and 10% human serum (complete medium). Cultures were gassed with a mixture of 3% O₂, 4% CO₂ and 93% N₂.

Antimalarial activity was assessed with an adaption of the 48-h sensitivity assay of Desjardins et al. (Antimicrob. Agents. Chemother., 1979,16, 710-718) using [³H]-hypoxanthine incorporation as an assessment of parasite growth. Stock drug solutions were prepared in 100% dimethylsulphoxide (DMSO) and diluted to the appropriate concentration using complete medium. Assays were performed in sterile 96-well microtitre plates, each plate containing 200 µl of parasite culture (2% parasitemia, 0.5% haematocrit) with or without 10 µl drug dilutions. Each drug was tested in triplicate and parasite growth was compared to control wells (which constituted 100 % parasite growth). After 24-h incubation at 37°C, 0.5 µCi hypoxanthine was added to each well. Cultures were incubated for a further 24 h before they were harvested onto filter-mats, dried for 1 h at 55°C and counted using a Wallac 1450 Microbeta Trilux Liquid scintillation and luminescence counter. IC₅₀ values were calculated by interpolation of the probit transformation of the log dose - response curve.

Several of these new dimers were shown to have remarkable activity against the K1 strain. For example, phosphate dimer PV40 is greater than 50 times more potent than the parent drug artemisinin and about 15 times more potent than the clinically used acetal artemether. Even more dramatic is the comparison with the 4-aminoquinoline chloroquine in this strain, where trioxanes PV37 and PV40 are between 400 to 900 times more potent. The data shown in Table 1 clearly demonstrate that trioxane dimers in this class are amongst the most potent antimalarials to have been tested against the K1 strain of *P. falciparum.*

**Table 1**

| | IC₅₀(nM) | Mean | S.D. |
|---|---|---|---|
| PV30 | 3.3 | | |
| | 1.6 | 2.6 | ±0.9 |
| | 2.9 | | |
| PV31 | 39.9 | | |
| | 43.3 | 42.2 | ±1.9 |
| | 43.3 | | |
| PV32 | 5.5 | | |
| | 5.2 | 4.6 | ±1.4 |
| | 3.0 | | |
| PV33 | 3.4 | | |
| | 1.3 | 1.8 | ±1.4 |
| | 0.7 | | |
| PV35 | 2.6 | | |
| | 3.1 | 2.4 | ±0.8 |
| | 1.6 | | |
| PV37 | 0.9 | | |
| | 0.4 | 0.5 | ±0.3 |
| | 0.3 | | |
| PV40 | 0.3 | | |
| | 0.1 | 0.2 | ±0.1 |
| | 0.2 | | |
| PV43 | 5.0 | | |
| | 2.1 | 2.9 | ±1.9 |
| | 1.5 | | |
| PV45 | 4.4 | | |
| | 2.6 | 2.7 | ±1.6 |
| | 1.2 | | |
| PV41 * | 2.6 | | |
| | 3.1 | 2.4 | ±0.8 |
| | 1.6 | | |
| Artemether | 4.5 | | |
| | 2.0 | 2.8 | ±1.4 |
| | 2.0 | | |
| Artemisinin | 12.3 | 12.3 | SE =±1.4 |
| Chloroquine | 190 | 190 | SE = ±8 |

According to a further aspect of the present invention there is provided a compound of general formula 11 as hereinbefore defined, or a pharmaceutically acceptable salt thereof, for use as a medicament.

Compounds of the present invention may be used particularly, but not exclusively, as medicaments for the treatment of malaria or cancer. Whilst the currently preferred use of peroxides is for treatment, it cannot be ruled out that these compounds would have a use in the prophylaxis of malaria.

A therapeutically effective non-toxic amount of a compound of general formula 11 as hereinbefore defined may be administered in any suitable manner, including orally, parenterally (including subcutaneously, intramuscularly and intravenously), or topically. The administration will generally be carried out repetitively at intervals, for example once or several times a day.

The amount of the compound of general formula 11 that is required in order to be effective as an antimalarial or anticancer agent for treating human or animal subjects will of course vary and is ultimately at the discretion of the medical or veterinary practitioner treating the human or animal in each particular case. The factors to be considered by such a practitioner, e.g. a physician, include the route of administration and pharmaceutical formulation; the subject's body weight, surface area, age and general condition; and the chemical form of the compound to be administered.

In daily treatment, for example, the total daily dose may be given as a single dose, multiple doses, e.g. two to six times per day, or by intravenous infusion for any selected duration.

The compound of general formula 11 may be presented, for example, in the form of a tablet, capsule, liquid (e.g. syrup) or injection.

While it may be possible for the compounds of general formula 11 to be administered alone as the active pharmaceutical ingredient, it is preferable to present the compounds in a pharmaceutical composition.

According to a further aspect of the present invention therefore there is provided a pharmaceutical composition containing a compound of general formula 11 as hereinbefore defined, or a pharmaceutically acceptable salt thereof, as an active ingredient.

Such pharmaceutical compositions for medical use will be formulated in accordance with any of the methods well known in the art of pharmacy for administration in any convenient manner. The compounds of the invention will usually be admixed with at least one other ingredient providing a compatible pharmaceutically acceptable additive, carrier, diluent or excipient, and may be presented in unit dosage form.

The carrier(s) must be pharmaceutically acceptable in the sense of being compatible with the other ingredients of the formulation and not deleterious to the recipient thereof.

The possible formulations include those suitable for oral, rectal, topical and parenteral (including subcutaneous, intramuscular and intravenous) administration or for administration to the lung or another absorptive site such as the nasal passages.

All methods of formulation in making up such pharmaceutical compositions will generally include the step of bringing the compound of general formula 11 into association with a carrier which constitutes one or more accessory ingredients. Usually, the formulations are prepared by uniformly and intimately bringing the compound of general formula 11 into association with a liquid carrier or with a finely divided solid carrier or with both and then, if necessary, shaping the product into desired formulations.

Formulations of the present invention suitable for oral administration may be presented as discrete units such as capsules, cachets, tablets or lozenges, each containing a predetermined amount of the compound of general formula 11; as a powder or granules; or a suspension in an aqueous liquid or non-aqueous liquid such as a syrup, an elixir, an emulsion or a draught. The compound of general formula 11 may also be presented as a bolus, electuary or paste.

A tablet may be made by compression or moulding, optionally with one or more accessory ingredients. Compressed tablets may be prepared by compressing, in a suitable machine, the compound of general formula 11 in a free-flowing form such as a powder or granules, optionally mixed with a binder, lubricant, inert diluent, surface active or dispersing agent. Moulded tablets may be made by moulding, in a suitable machine, a mixture of the powdered compound of general formula 11 with any suitable carrier.

A syrup may be made by adding the compound of general formula 11 to a concentrated, aqueous solution of a sugar, for example sucrose, to which may be added any desired accessory ingredient. Such accessory ingredient(s) may include flavourings, an agent to retard crystallisation of the sugar or an agent to increase the solubility of any other ingredient, such as a polyhydric alcohol, for example glycerol or sorbitol.

Formulations for rectal administration may be presented as a suppository with a usual carrier such as cocoa butter.

Formulations suitable for parental administration conveniently comprise a sterile aqueous preparation of the compound of general formula 11 which is preferably isotonic with the blood of the recipient.

In addition to the aforementioned ingredients, formulations of this invention, for example ointments, creams and the like, may include one or more accessory ingredients, for example a diluent, buffer, flavouring agent, binder, surface active agent, thickener, lubricant and/or a preservative (including an antioxidant) or other pharmaceutically inert excipient.

The compounds of this invention may also be made up for administration in liposomal formulations which can be prepared by methods well-known in the art.

A further aspect of the present invention provides the use of a compound of general formula 11 as hereinbefore defined, or a pharmaceutically acceptable salt thereof, in the manufacture of a medicament for the treatment or prophylaxis of malaria.

A further aspect of the present invention provides the use of a compound of general formula 11 as hereinbefore defined, or a pharmaceutically acceptable salt thereof, in the manufacture of a medicament for the treatment of cancer.

It has further surprisingly been found that exposure of tumour cells to iron potentiates the anticancer effect of the compounds of the present invention. This finding is supported by the observation that cultured cancer cell lines were more readily destroyed by compounds of the present invention following exposure to culture medium having an elevated iron concentration.

According to a further aspect of the present invention therefore there is provided a product containing a first compound of general formula 11 as hereinbefore defined, or a pharmaceutically acceptable salt thereof, and a second, iron-containing, compound as a combined preparation for simultaneous, separate or sequential use in the treatment of cancers.

Preferably, the first and second compounds are used sequentially, the second, iron-containing compound being used first.

The first compound may be presented in any of the forms described above. Administration of the first compound may be in any suitable manner, including intravenously, intraarterially, intralesionally, topically, intracavitarily or orally. Any suitable dosage of the compound may be used. Preferably, a dosage within the range of 0.1 to 500mg/kg body weight is used, more preferably within the range of 0.5 to 300mg/kg body weight, such as 1 to 50 mg/kg body weight.

With regard to the iron-containing compound, this may take any suitable form. Preferred agents for enhancing intracellular iron levels for use in the present invention include pharmaceutically acceptable iron salts and iron complexes. Iron salts useful in the present invention include ferrous fumarate, ferrous sulphate, ferrous carbonate, ferrous citrate, ferrous gluconate, ferrous lactate and ferrous maleate. Iron complexes useful in the present invention include ferrocholinate, ferroglycine sulphate, dextran iron complex, peptonized iron, iron sorbitex, saccharated iron, iron complexed with iron binding proteins and glycoproteins such as the holoferritins and holotransferrins.

The iron-containing compound may be presented in any of the forms described above in relation to the compound of general formula 13. Administration of the iron-containing compound may be achieved via any of the possible routes of administration of the first compound. The first and second compounds may be administered via the same or different routes.

The iron-containing compound may be used at any appropriate dosage, but is preferably used at a dosage within the range of 0.01 to 1000 mg iron/kg body weight.

The product of the invention may further comprise one or more other agents known to be useful in the treatment of tumours. Such agents may include, for example, androgen inhibitors, antiestrogens, antimetabolites and cytotoxic agents.

Compounds of general formula 11, and pharmaceutically acceptable salts thereof, are useful in the treatment of malaria.

In terms of suitable dosages for the treatment of malaria, the preferred amount of compounds of the present invention is between 10 mg to 5 g, preferably 50 to 1000 mg, administered over a period of 2-5 days, alone or in combination with other antimalarial drugs, such as, for example, the class II blood schizonticides or halofantrine (Looaeesuwan, Am. J. Trop.Med., 1999,60,238).

Compounds of general formula 11, and pharmaceutically acceptable salts thereof, are useful in the treatment of cancer.

Treatments may further comprise the simultaneous, separate or sequential administration to the said animal an effective amount of an iron-containing compound as hereinbefore described.

The invention will now be illustrated further by the following non-limiting examples.

### Examples

### Dihydroartemisinin 10-α-benzoate (13)

Benzoyl chloride (3.17ml, 27.29 mmol) was added to a solution of Dihydroartemisinin (5.0g,17.61 mmol) in anhydrous dichchloromethane (54 ml) and anhydrous pyridine (9 ml) at 0°C. The mixture was allowed to stir at room temperature for 16 hours. The reaction mixture was then partitioned between 7% citric acid (50 ml) and ethyl acetate (2 x 50 ml). The organic phase was dried over MgSO₄, filtered and concentrated under reduced pressure. Purified by silica gel chromatography using ethyl acetate/ⁿhexane (10/90) as the eluent to give the product as a white crystalline solid. (90%, mp 111-112° C).

¹H NMR(300MHz, CDCl₃) δ 8.12(m, 2H, ArH), 7.57(m,1H, ArH), 7.44(m, 2H, ArH), 6.00(d, J=9.6Hz, 1H), 5.24(s, 1H), 2.75(m, 1H),2.39(dt, J=14.4, 3.9Hz, 1H), 1.42(s, 3H), 0.98(d, J=6.0Hz, 3H),0.92(d, J=7.2Hz, 3H); ¹³C NMR(75MHz, CDCl₃) ppm 165.47, 133.37, 130.22, 129.8, 128.4, 104.5, 92.6, 91.64, 80.2, 51.67, 45.35, 37.24, 36.23, 34.1, 31.9, 25.8, 24.5, 22.0, 20.1, 12.1; MS(CI, NH₃) 406([M+NH₄]⁺, 100), 360(52), 343(45), 325(31), 221(100), 207(50),105(9); Calculated for C₂₂H₃₂NO₆[M+NH₄]⁺ requires 406.22296 found 406.22403; Anal.C₂₂H₂₈O₆ requires C 68.04%, H 7.22%, found C 67.63%, H 7.30%.

### 10β-Allyldeoxoartemisinin (12a)

A solution of dihydroartemisinin 10α-benzoate (1.1g, 2.8 mmol) in anhydrous 1,2-dichloroethane (11 ml) was added via cannula into a mixture of allyltrimethylsilane (2.2 ml,13.8 mmol) and ZnCl₂ (0.5g, 3.67mmol) in anhydrous 1,2-dichloroethane (11 ml), which was stirred over activated 4A⁰ molecular sieves under nitrogen at 0° C for 2 hour and then allowed to warm to RT. The mixture was diluted with ethyl acetate, washed with 5% citric acid, saturated NaHCO₃ and brine. The organic layer was dried over MgSO₄, filtered and concentrated under reduced pressure. The crude product was purified by silica gel chromatography with ethyl acetate/ⁿhexane (10/90) as eluent to give the product as a white solid (90%, mp 76-78° C).

¹H NMR (300 MHz, CDCl₃) δ 5.92(m, 1H), 5.32(s, 1H), 5.08(m, 2H), 4.30(m, 1H), 2.67(sex, 1H), 2.44-2.17(m, 3H), 1.40(s, 3H), 0.95(d, J=6.0Hz, 3H), 0.88(d, J=7.5Hz, 3H); ¹³C NMR(75MHz, CDCl₃) ppm 136.6, 116.1, 103.1, 89.2, 81.1, 74.1, 52.1, 44.1, 37.1, 36.6, 34.4, 34.2, 30.16, 25.98, 24.8, 24.6, 20.1, 12.8; MS(CI, NH₃) 326([M+NH₄]⁺, 20), 309([M+H]⁺, 10), 263(100), 221(70), 205(100), 162(80), 95(15), 58(20); Calculated for C₁₈H₂₉O₄[M+H]⁺ requires309.20659 found 309.20620; Anal. C₁₈H₂₈O₄ requires C 70.09%, H 9.15%, found C 70.03%, H 9.15%.

### 10β-(2-hydroxyethyl)-deoxoartemisinin (9b)

A solution of 10β-Allyldeoxoartemisinin (0.6g, 1.94 mmol) in anhydrous dichloromethane (DCM) (150 ml) was flushed with N₂ and then subjected to a steady stream of O₃ at -78° C until the solution became saturated with O₃ and appeared blue. Residual O₃ was flushed of from the mixture with N₂ and the solvent was removed under reduced pressure. THF/MeOH (150 ml, 9:1) was added and the solution was treated with excess sodium borohydride (4.00g) at 0° C for 4 hours. The resultant mixture was concentrated under reduced pressure and water and CHCl₃ were added. The organic layer was dried over MgSO₄, filtered and the solvent was removed under reduced pressure. Purification by silica gel chromatography using ethyl acetate/ⁿhexane (40/60) as the eluent, gave the product as a white solid (68%,mp 104-106° C).

¹H NMR (300MHz, CDCl₃) δ 5.35(s, 1H), 4.44(m, 1H), 3.83(q, 2H), 2.65(sex, 1H), 2.54(m, 1H), 2.32(dt, J=13.5, 4.0 Hz), 1.4(s, 3H), 0.96(d, J=5.91Hz, 3H), 0.86(d, J=7.55Hz, 3H); ¹³C NMR (75MHz, CDCl₃) ppm 103.2, 89.3, 81, 74.9, 62.6, 52.2, 44.1, 37.4, 3.4, 31.6, 30.3, 25.9, 24.6, 20, 12.7; MS (CI, NH₃) 330([M+NH₄]⁺, 30), 313([M+H]⁺, 10), 295(100), 267(52), 253(100), 237(40), 209(35), 183(27). Calculated for C₁₇H₂₉O₅ [M+H]⁺ requires 313.20150 found 313.20185; Anal. C₁₇H₂₈O₅ requires C 65.39%, H 9.04% found C 65.53%, H 9.17%.

### General Procedure 1: Synthesis of Bisester Dimers

4-(Dimethylamino) pyridine (0.05g, 0.4 mmol) and anhydrous DCM (8 ml) were added to a flask charged with vacuum-dried trioxan alcohol (0.11g, 0.35mmol). This mixture was cooled to 0° C, and a bis-acid chloride was added. The reaction was stirred overnight and the crude mixture was directly absorbed onto silica gel and loaded onto a silica column. Chromatography (ethyl acetate/ⁿhexane) gave the desired dimer.

### PV 30

### 10β-(2-Hydroxyethyl)-deoxoartemisinin bis-ester (1,2)-dimer

Using the general procedure, trioxane dimer (PV 30) was prepared and purified by silica gel chromatography using ethyl acetate / ⁿhexane (40/60) as the eluent (61%, Mp 63-65° C)

¹H NMR (400MHz, CDCl₃) δ 7.74(dd, J=5.66, 3.32 Hz, 2H), 7.5(dd, J=5.66, 3.31Hz, 2H), 5.33(s, 2H), 4.56(m, 2H), 4.40(m, 4H), 2.7(sex, J=6.7Hz, 2H), 2.31(dt, J=14.0, 4.05Hz, 2H), 1.36(s, 6H), 0.95(d, J=6.13Hz, 6H), 0.88(d, J=7.54Hz, 6H); ¹³C NMR (100MHz, CDCl₃) ppm 167.8, 132.7, 131.2, 129.3, 103.5, 89.4, 81.4, 72.0, 64.0, 52.7, 44.6, 37.8, 36.9, 34.8, 30.5, 29.2, 26.4, 25.18, 25.13, 20.5, 13.2; MS (FAB) 755([M+H]⁺, 70), 777([M+Na]⁺, 25), 721(100), 704(62), 664(65), 483(42), 443(48), 77(35), 55(55); Anal. C₄₂H₅₈O₁₂ requires C 66.80%, H 7.75% found C 66.36% H 7.90%.

### PV 31

### 10β-(2-hydroxyethyl)-deoxoartemisininbis-ester(1,3)-dimer

Using the general procedure, trioxane dimer (PV 31) was prepared and purified by silica gel chromatography using ethyl acetate / ⁿhexane (50/50) as the eluent (58%, mp168-169° C)

¹H NMR (300MHz, CDCl₃) δ 8.69(d, 1H, Ar-H), 8.23(dd, J=7.83, 1.65Hz, 2H, Ar-H), 7.51(t, J=7.83, 1H, Ar-H), 5.36(s, 2H), 4.59(m, 2H), 4.45(m, 4H), 2.74(sex, 2H), 2.33(dt, J=13.0, 3.98Hz, 2H), 1.36(s, 6H), 0.95(d, J=6.05Hz, 6H), 0.91(d, J=7.56Hz, 6H); ¹³C NMR (75MHz, CDCl₃) ppm 165.8, 133.8, 130.9, 130.7, 128.5, 103.2, 89.0, 81.0, 71.6, 63.5, 52.3, 44.3, 37.5, 36.5, 34.5, 30.1, 29.0, 26.0, 24.8, 24.7, 20.1, 12.8; MS (FAB) 777([M+Na]⁺, 25) 755([M+H]⁺, 22), 737(45), 721(100), 709(60), 703(100), 664(95), 443(87), 425(81), 295(100), 76(35), 54(62); Anal. C₄₂H₅₈O₁₂ requires C 66.80 %, H 7.75% found C 65.75%, H 7.64%.

### PV 32

### 10-β-(2-Hydroxyethyl)-deoxoartemisinin bis-ester(1,4)-dimer

Using the general procedure, trioxane dimer (PV 32) was prepared and purified by silica gel chromatography using ethyl acetate / ⁿhexane (50/50) as the eluent (75%, mp 66-68° C).

¹H NMR (400MHz, CDCl₃) δ 8.1(s, 4H, Ar-H), 5.35(s, 2H), 4.6(m, 2H), 4.46(m, 4H), 2.68(sex, 2H), 2.3(dt, 2H), 1.34(s, 6H), 0.96(d, J=5.92, 6H), 0.9(d, J=7.51, 6H); ¹³C NMR (100MHz, CDCl₃) ppm 166, 13.5, 129.9, 103, 89.6, 81.4, 71.9, 71.7, 52.6, 44.5, 37.8, 36.9, 34.8, 30.5, 29.4, 26.3, 25.2, 20.5, 13; MS (Anal. C₄₂H₅₈O₁₂ requires C 66.80% H 7.75% found C 66.80% H 7.83%.

### PV33

Using the General Procedure 1, trioxane dimer (PV 33) was prepared and purified by silica gel chromatography using ethyl acetate / ⁿhexane (50/50) as the eluent (66%, mp107° C).

¹H NMR (300MHz, CDCl₃) δ 5.3(s, 2H), 4.3(m, 4H), 4.19(m, 2H), 2.67(sex, 2H) 2.62(s, 4H), 2.32(dt, J=13.5, 3.9, 2H), 1.39(s, 6H), 0.94(d, J=6Hz, 6H), 0.86(d, J=7.5, 6H); ¹³C NMR (75MHz, CDCl₃) ppm 172.3, 103.2, 89, 81, 71.8, 62.9, 52.3, 44.3, 37.4, 36.5, 34.4, 30, 29.2, 28.7, 26, 24.8, 24.7, 20.1,12.8; MS (FAB) 707 ([M+H]⁺, 6), 729([M+Na]⁺, 8), 673(23), 616(18), 423(16), 379(15), 293(77), 277(82), 249(100), 209(75); Anal C₃₈H₅₈O₁₂ requires C 64.55% H 8.27% found C 63.95% H 8.15.

### PV35

Using the General Procedure 1, trioxane dimer (PV 35) was prepared and purified by silica gel chromatography (ethyl acetate / ⁿhexane (50/50) (60%, mp 41-43° C)¹H NMR (400MHz, CDCl₃) δ 5.3(s, 2H), 4.3(m, 4H), 4.19(m, 2H), 2.6(sex, 2H), 2.36(t, 4H), 1.4(s, 6H), 0.96(d, J=5.9, 6H), 0.86(d, J=7.52, 6H); ¹³C NMR (100MHz, CDCl₃) ppm 173.2, 103.6, 89.3, 81.4, 72.3, 63, 52.6, 44.6, 37.8, 36.8, 34.8, 33.7, 30.4, 29, 26.4, 25.1, 25.1, 20.5, 20.4, 13.3;

### PV 36

### 10β-(2-trimethylsillyloxyethyl)-deoxoartemisinin

Triethylamine (0.07 ml, 0.5 m mol) and trimethylsilylchloride (0.08 ml, 0.63 mmol) was added to a solution of trioxane alcohol (0.1g, 0.32 mmol) in anhydrous DCM (5 ml) under N₂. The mixture was allowed to stir at 30° C for 45 minute and then filtered through celite. Purification by silica gel chromatography using ethyl acetate / DCM (10/90) as the eluent, give the product as the colourless syrup (96%).

¹H NMR (400MHz, CDCl₃) δ 5.19(s, 1H), 4.05(m, 1H), 3.7(m, 1H), 3.51(m, 1H), 2.67(sex, 1H), 2.26(dt, J=13.1,3.97 Hz, 1H), 1.4(s, 3H), 0.94(d, J=6 Hz, 3H), 0.84(d, J=7.53 Hz, 3H), 0.1(s, 9H); ¹³C NMR (100MHz, CDCl₃) ppm 103.9, 89.0, 81.5, 73.5, 61.5, 53.0, 45.0, 37.8, 36.8, 34.7, 32.8, 30.4, 26.6, 25.24, 25.13, 20.7, 13.8, 0.06.

### General Procedure 2: Synthesis of Bisether Dimers

TMSOTf (10 µl) was added to a mixture of appropriate dialdehyde and trimethylsilyloxytrioxanealcohol (0.1 g, 0.3 mmol) in anhydrous DCM (3 ml) at -78° C. After stirring at -78° C for one hour, triethylsilane (0.04 ml, 0.3 mmol) was added and the mixture was stirred at -78 to -30° C for four hours. The reaction mixture was diluted with diethylether (3 ml), washed with saturated NaHCO₃, dried over MgSO₄. The organic extracts were removed under reduced pressure and the product purified by chromatography.

### PV 41

Using General Procedure 2 (PV 41) was prepared and purified by silica gel chromatography using ethyl acetate / DCM (10/90) as the eluent (74%, mp 46-48 ° C).

¹H NMR (400MHz, CDCl₃) δ 7.33(s, 4H, Ar-H), 5.31(s, 2H), 4.5(s, 4H), 4.22(m, 2H), 3.7(m, 2H), 3.69(m, 2H), 2.7(sex, 2H), 2.3(dt, J=13.1, 3.98, 2H), 1.4(s, 6H), 0.94(d, J=5.95, 6H), 0.86(d, J=7.53, 6H); ¹³C NMR (100MHz, CDCl₃) ppm 138, 128, 103.7, 89, 81, 73.4, 73.27, 69, 52.8, 45, 37.8, 36.9,34.9, 30.4, 30.1, 26.5, 25, 20.5, 13.5; MS (FAB) 1452([2m+H]⁺, 35), 725([M-H]⁺, 14), 693(10), 381(30), 295(51), 209(100), 277(50), 237(64), 136(33), 105(52), 77(35); Anal C₄₂H₆₂O₁₀ requires C 69.38%, H 8.60% found C

### PV 43

Using General Procedure 2, trioxane dimer (PV 43) was prepared and purified by silica gel chromatography using ethyl acetate / DCM (15/85) as the eluent (58%).

¹H NMR (400MHz, CDCl₃) δ 7.3(m, Ar-H, 4H), 5.31(s, 2H), 4.51(s, 4H), 4.26(m, 2H), 3.72(m, 2H), 3.6(q, 2H), 2.72(sex, 2H), 2.31(dt, J=15.0, 3.98 Hz, 2H), 1.4(s, 6H), 0.95(d, J=6.0 Hz, 6H), 0.86(d, J=7.48 Hz, 6H); ¹³C NMR (100MHz, CDCl₃) ppm 139, 128.7, 127.4, 127, 103.6, 89.3, 81.4,73.5, 73.2, 69.3, 52.8, 45, 37.8, 37, 34.8, 30.4, 30, 25, 20.5,13.5; MS(FAB)

Anal C₄₂H₆₂O₁₀ requires C 69.38%, H 8.60% found C 69.33%, H 8.68%.

### PV 45

Using General Procedure 2, trioxane dimer (PV 45) was prepared and purified by silica gel chromatography using ethyl acetate / ⁿhexane (30/70) as the eluent (53%).

¹H NMR (400MHz, CDCl₃) δ 7.39(dd, J=3.4,1.98Hz, 2H), 7.26(dd, J=3.49,2.0Hz, 2H,Ar-H), 5.31(s, 2H), 4.58(s, 4H), 4.24(m, 2H), 3.73(m, 2H), 3.59(q, 2H), 2.72(sex, 2H), 2.33(dt, J=15, 3.9Hz, 2H), 1.4(s, 6H), 0.95(d, J=6Hz, 6H), 0.87(d, J=4.59, 6H); ¹³C NMR (100MHz, CDCl₃) ppm 136.9, 128.8, 127.9, 103.6, 89, 81.4, 73.3, 71, 69, 52.8, 45, 37.8, 36, 35, 30.4, 30.1, 26.5, 25, 20.5, 13; MS (FAB) 727 ([M+H]⁺, 4), 429(5), 191(10), 121(20), 105(90), 77(40), 43(100); Anal C₄₂H₆₂O₁₀ requires C 69.38%, H 8.60% found C 69.18%, H 8.63%.

### General Procedure 3: Synthesis of Bis phosphate Ester Dimers

Sodium hexamethyldisilazide (NaHMDS 1.0 M in THF, 0.33 ml, 0.32 mmol) was added, via syringe to a solution of trioxane alcohol (0.1g, 0.32 mmol) in THF (7 ml) at 0° C. The resulting mixture was stirred for 10 minute at 0° C and the appropriate dichlorophosphate (0.024 ml, 0.16 mmol) was added. The reaction was kept at 0° C for 2 hour and was then warmed to RT and stirred for 40 minutes. The mixture was then cooled back to 0° C, quenched with water. The organic layer was extracted three times with ether, the combined organic portions were washed with satd NaCl and dried over MgSO₄. The organic extracts were removed under reduced pressure and the product purified by chromatography.

### PV 37

Using General Procedure 3, trioxane dimer (PV 37) was prepared and purified by column chromatography using ethyl acetate / ⁿhexane (70/30) as the eluent (49%).

¹H NMR (400MHz, CDCl₃) δ 7.32(t, 2H, Ar-H), 7.24(t, 2H, Ar-H), 7.14(t, 1H, Ar-H), 5.28(s, 2H), 4.38(m, 2H), 4.28(m, 4H), 2.67(sex, 2H), 2.32(dt, J=13.0, 3.98 Hz, 2H), 1.39(s, 6H), 0.95(d, J=5.87, 6H), 0.84(d, J=7.36, 3H) and 0.82(d, J=7.64,3H); ¹³C NMR (100MHz, CDCl₃) ppm 130, 125, 120.5, 103.5, 89.3, 81.0, 71.5, 67.0, 52.7, 44.6, 37.8, 36.9, 34.8, 30.9, 30.3, 26.4, 25, 20.5 and 13.2;

### PV 40

Using General Procedure 3, trioxane dimer (PV 40) was prepared and purified by column chromatography using ethyl acetate / ⁿhexane (70/30) as the eluent (58%, mp 48-50 ° C).

¹H NMR (400MHz, CDCl₃) δ 5.3(s, 2H), 4.29(m, 4H), 4.16(m, 2H), 3.76(d, J=11.1 Hz, 3H), 2.68(sex, 2H), 2.28(dt, J=13.1,3.96Hz), 1.40(s, 6H), 0.96(d, J=6.0Hz, 6H) and 0.87(d, J=7.48 Hz, 6H); ¹³C NMR (100MHz, CDCl₃) ppm 103.5, 89.3, 81.3, 71.6, 67, 52.7, 44.6, 37.8, 36.9, 34.8, 30.9, 30.3, 26.3, 25.1, 20.5 and 13.2; MS (FAB) 701([M+H]⁺,10), 307(8), 237(8), 191(25), 154(72), 136(68), 107(46), 77(52); MS(FAB) 701([M+H]⁺,10), 307(8),237(8), 191(25), 154(72), 136(68), 107(46),77(52); Anal. C₃₅H₅₇O₁₂P requires C 59.97%, H 8.20% found C 60.26% H 8.26%.

It is to be understood that the present invention is not intended to be restricted to the details of the above examples and embodiments, which are described by way of example only.

## Claims

1. A compound of the following general formula: 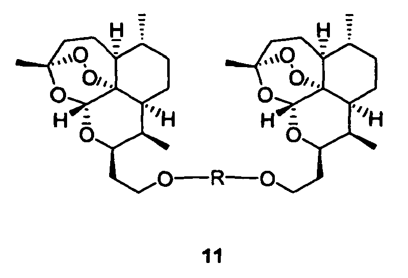 or a pharmaceutically acceptable salt thereof,
in which R is selected from the group comprising optionally substituted alkyl, aryl, bisalkyl or bisaryl ester, bisalkyl or bisaryl ether, acetal, ketal, boronate, silyl ether, carbamate, carbonate, sulphate, sulphonate, phosphate and phosphonate.

2. A compound according to claim 1, wherein R comprises an optionally substituted alkyl or aryl group of 1-20 carbon atoms.

3. A compound according to claim 2, wherein R comprises an optionally substituted alkyl or aryl group of 1-12 carbon atoms.

4. A compound according to any of claims 1 to 3, wherein R comprises an alkyl or aryl group having a substituent selected from primary, secondary and tertiary amines; halogen-containing groups; alcohols and derivatives thereof; and carboxylic acids and derivatives thereof.

5. A compound according to claim 4, wherein R is an alkyl or aryl group having a substituent selected from primary, secondary and tertiary amines.

6. A compound according to claim 1, wherein R is phosphate.

7. A compound according to claim 1, wherein R is selected from the following groups:

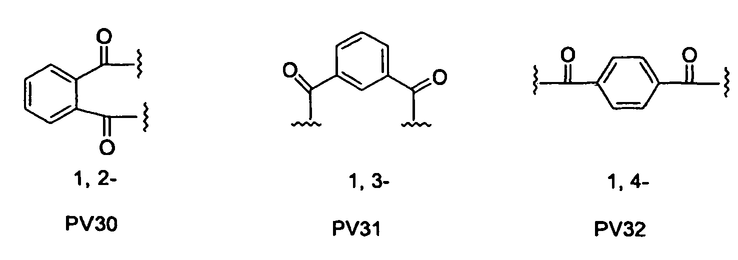

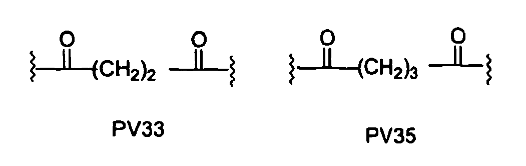

8. A compound according to claim 1, wherein R is selected from the following groups:

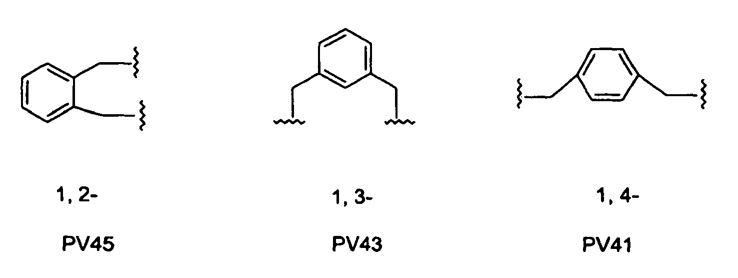

9. A compound according to claim 1, wherein R is selected from the following groups:

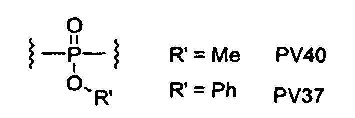

10. A compound according to claim 1, wherein R is selected from the following groups:

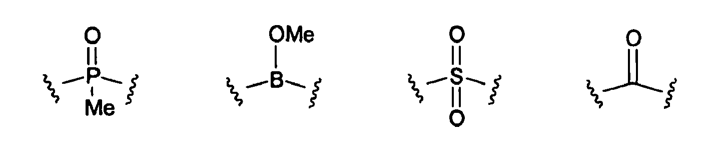

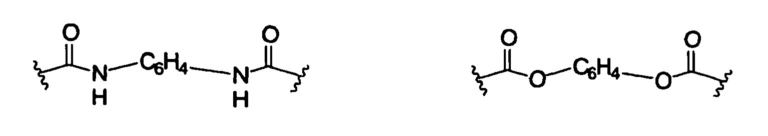

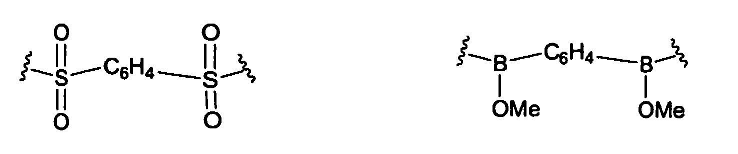

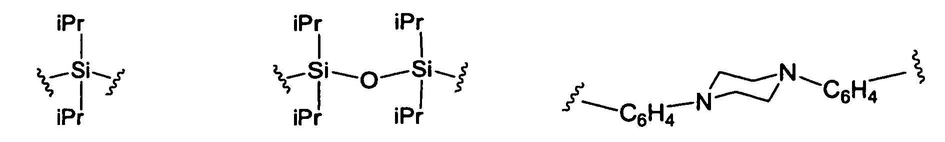

11. A process for the production of a compound according to claim 1, wherein the process comprises the reaction of the C-10 benzoate of dihydroartemesinin with allyltrimethylsilane in the presence of a weak Lewis acid to form 10β-allyldeoxoartemesinin, and ozonolysis of the 10β-allyldeoxoartemesinin and subsequent reduction of the ozonide to form 10β-(2-hydroxyethyl) deoxoartemisinin.

12. A process according to claim 11, wherein the weak Lewis acid is zinc chloride.

13. A process according to either of claims 11 or 12, wherein crystalline C-10-α-benzoate is reacted with allyltrimethylsilane in the presence of zinc chloride in anhydrous dichloroethane as a solvent.

14. A process according to any of claims 11 to 13, wherein the reaction is carried out in the presence of a dehydrating agent.

15. A process according to claim 14, wherein the dehydrating agent comprises 4Å molecular sieves.

16. A process according to any of claims 11 to 15, wherein the reaction is carried out under an inert atmosphere.

17. A process according to any of claims 11 to 16, wherein the reaction is carried out at a temperature within the range of -10°C to 0°C.

18. A process according to any of claims 11 to 17, wherein the reaction time is 4 hours.

19. A process according to any of claims 11 to 18, wherein the ozonide is reduced *in situ*.

20. A process for the preparation of a trioxane derivative as claimed in claim 1, comprising treating 10β-(2-hydroxyethyl)-deoxoartemisinin with an acid chloride and a catalytic quantity of 4-(dimethylamino) pyridine.

21. A process according to claim 20, wherein two equivalents of 10β-(2-hydroxyethyl)-deoxoartemisinin are treated with one equivalent of acid chloride.

22. A process according to either of claims 20 or 21, wherein the trioxane derivative comprises a compound according to claim 7.

23. A process for the preparation of a trioxane derivative as claimed in claim 1, comprising the mild bis-reductive etherification of the following *O*-trimethylsilyl ether derivative: 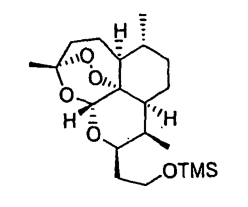 using a bis-aldehyde in the presence of a Lewis acid and triethylsilane as a reducing agent.

24. A process according to claim 23, wherein the Lewis acid is TMSOTf.

25. A process according to claim 24, wherein 0.2 equivalents of TMSOTf are used.

26. A process according to any of claims 23 to 25, wherein the reaction temperature is controlled at temperatures of from 78°C to -30°C.

27. A process according to any of claims 23 to 26, wherein the trioxane derivative comprises a compound according to claim 8.

28. A process according to any of claims 23 to 27, wherein the *O*-trimethylsilyl ether derivative is prepared from 10β-(2-hydroxyethyl)-deoxoartemisinin.

29. A process for the preparation of a trioxane derivative as claimed in claim 1, comprising treatment of 10β-(2-hydroxyethyl)-deoxoartemisinin with sodium hexamethyldisilazide and a dichlorophosphate.

30. A process according to claim 29, wherein two equivalents of 10β-(2-hydroxyethyl)-deoxoartemisinin are treated with one equivalent of sodium hexamethyldisilazide and one equivalent of a dichlorophosphate, in anhydrous THF.

31. A process according to either of claims 29 or 30, wherein the trioxane derivative comprises a compound according to claim 9.

32. A compound according to any of claims 1 to 10, or a pharmaceutically acceptable salt thereof, for use as a medicament.

33. A compound according to claim 32, wherein the medicament is a medicament for the treatment of malaria or cancer.

34. A pharmaceutical composition containing a compound according to any of claims 1 to 10, or a pharmaceutically acceptable salt thereof, as an active ingredient.

35. The use of a compound according to any of claims 1 to 10, or a pharmaceutically acceptable salt thereof, in the manufacture of a medicament for the treatment or prophylaxis of malaria.

36. The use of a compound according to any of claims 1 to 10, or a pharmaceutically acceptable salt thereof, in the manufacture of a medicament for the treatment of cancer.

37. A product containing a first compound according to any of claims 1 to 10, or a pharmaceutically acceptable salt thereof, a second, iron-containing, compound as a combined preparation for simultaneous, separate or sequential use in the treatment of cancers.

38. A product according to claim 37, wherein the first and second compounds are for use sequentially, the second, iron-containing, compound being used first.

39. A product according to either of claims 37 or 38, wherein the second, iron-containing, compound is an iron salt selected from ferrous fumarate, ferrous sulphate, ferrous carbonate, ferrous citrate, ferrous gluconate, ferrous lactate and ferrous maleate.

40. A product according to either of claims 37 or 38, wherein the second, iron-containing, compound is an iron complex selected from ferrocholinate, ferroglycine sulphate, dextran iron complex, peptonized iron, iron sorbitex, saccharated iron, iron complexed with an iron binding protein, and iron complexed with a glycoprotein.

41. A product according to any of claims 37 to 40, wherein the product further comprises one or more other agents known to be useful in the treatment of tumours.

42. A product according to claim 41, wherein the one or more other agents are selected from androgen inhibitors, antiestrogens, antimetabolites and cytotoxic agents.

## Patentansprüche

1. Verbindung der folgenden allgemeinen Formel: 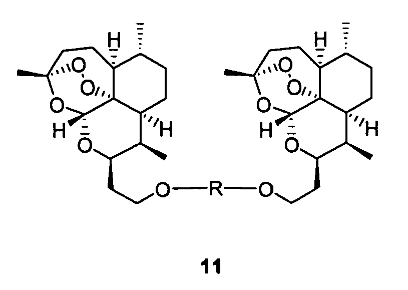 oder ein pharmazeutisch akzeptables Salz davon,
wobei R ausgewählt ist aus der Gruppe bestehend aus optional substituiertem Alkyl-, Aryl-, Bisalkyl- oder Bisarylester, Bisalkyl- oder Bisarylether, Acetal, Ketal, Boronat, Silylether, Carbamat, Carbonat, Sulfat, Sulfonat, Phosphat und Phosphonat.

2. Verbindung nach Anspruch 1, wobei R eine optional substituierte Alkyl- oder Arylgruppe mit 1-20 Kohlenstoffatomen umfasst.

3. Verbindung nach Anspruch 2, wobei R eine optional substituierte Alkyl- oder Arylgruppe mit 1-12 Kohlenstoffatomen umfasst.

4. Verbindung nach einem der Ansprüche 1 bis 3, wobei R eine Alkyl- oder Arylgruppe mit einem Substituenten, der ausgewählt ist aus Primär-, Sekundär- und Tertiäraminen, halogenhaltige Gruppen, Alkohole und Derivate davon und Carbonsäuren und Derivate davon umfasst.

5. Verbindung nach Anspruch 4, wobei R eine Alkyl- oder Arylgruppe mit einem Substituenten ist, der ausgewählt ist aus Primär-, Sekundär- und Tertiäraminen.

6. Verbindung nach Anspruch 1, wobei R Phosphat ist.

7. Verbindung nach Anspruch 1, wobei R aus den folgenden Gruppen ausgewählt ist:

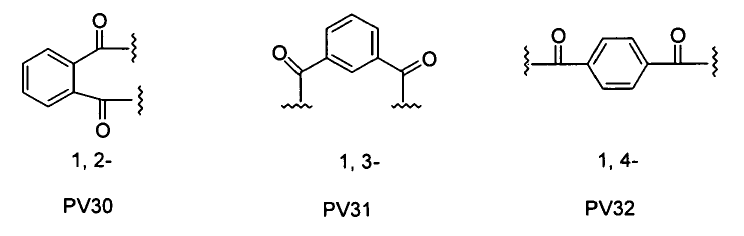

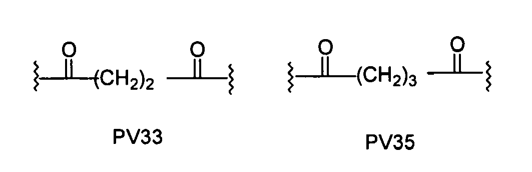

8. Verbindung nach Anspruch 1, wobei R aus den folgenden Gruppen ausgewählt ist:

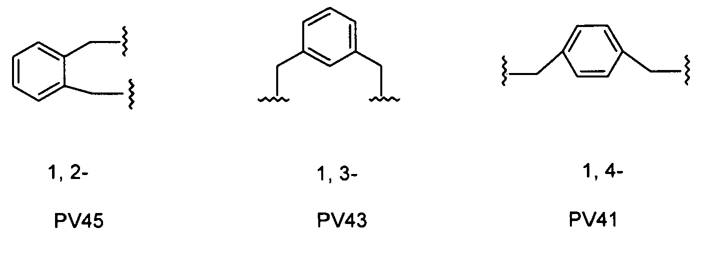

9. Verbindung nach Anspruch 1, wobei R aus den folgenden Gruppen ausgewählt ist:

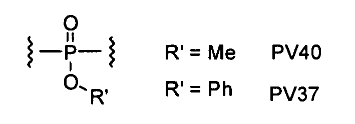

10. Verbindung nach Anspruch 1, wobei R aus den folgenden Gruppen ausgewählt ist:

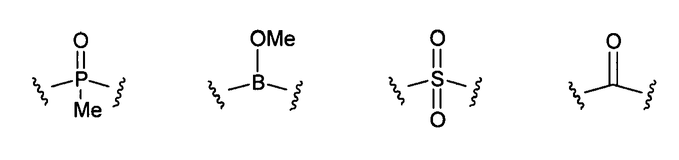

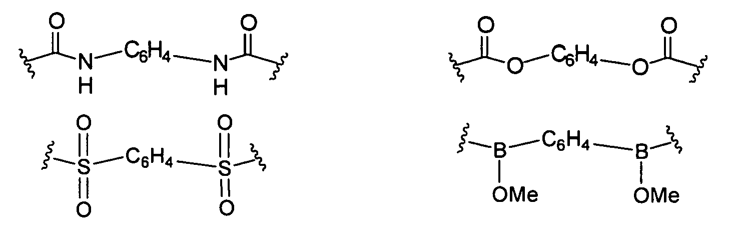

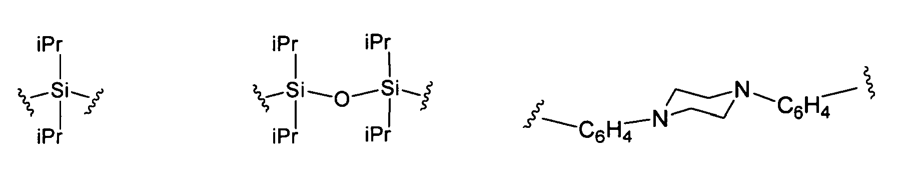

11. Verfahren zur Herstellung einer Verbindung nach Anspruch 1, wobei das Verfahren die Reaktion des C-10 Benzoats von Dihydroartemisinin mit Allyltrimethylsilan in Anwesenheit einer schwachen Lewis-Säure, um 10β-Allyldesoxoartemisinin zu bilden, sowie eine Ozonolyse des 10β-Allyldesoxoartemisinins und eine anschließende Reduktion des Ozonids zur Bildung von 10β-(2-Hydroxyethyl) desoxoartemisinin beinhaltet.

12. Verfahren nach Anspruch 11, wobei die schwache Lewis-Säure Zinkchlorid ist.

13. Verfahren nach Anspruch 11 oder 12, wobei kristallines C-10-α-Benzoat mit Allyltrimethylsilan in Anwesenheit von Zinkchlorid in wasserfreiem Dichlorethan als Lösungsmittel zur Reaktion gebracht wird.

14. Verfahren nach einem der Ansprüche 11 bis 13, wobei die Reaktion in Anwesenheit eines Dehydratisierungsmittels stattfindet.

15. Verfahren nach Anspruch 14, wobei das Dehydratisierungsmittel 4Å Molekularsiebe umfasst.

16. Verfahren nach einem der Ansprüche 11 bis 15, wobei die Reaktion unter einer inerten Atmosphäre stattfindet.

17. Verfahren nach einem der Ansprüche 11 bis 16, wobei die Reaktion bei einer Temperatur im Bereich von -10°C bis 0°C stattfindet.

18. Verfahren nach einem der Ansprüche 11 bis 17, wobei die Reaktionszeit 4 Stunden beträgt.

19. Verfahren nach einem der Ansprüche 11 bis 18, wobei das Ozonid *in situ* reduziert wird.

20. Verfahren zur Herstellung eines Trioxanderivats nach Anspruch 1, das das Behandeln von 10β-(2-Hydroxyethyl)-desoxoartemisinin mit einem Säurechlorid und einer katalytischen Menge von 4-(Dimethylamino) pyridin beinhaltet.

21. Verfahren nach Anspruch 20, wobei zwei Äquivalente von 10β-(2-Hydroxyethyl)-desoxoartemisinin mit einem Äquivalent von Säurechlorid behandelt werden.

22. Verfahren nach einem der Ansprüche 20 oder 21, wobei das Trioxanderivat eine Verbindung nach Anspruch 7 umfasst.

23. Verfahren zur Herstellung eines Trioxanderivats nach Anspruch 1, das eine milde bisreduktive Veretherung des folgenden *O*-Trimethylsilyletherderivats: 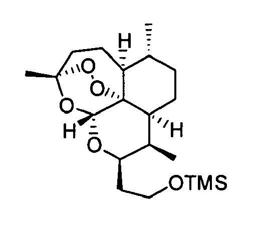 unter Verwendung eines Bisaldehyds in Anwesenheit einer Lewis-Säure und Triethylsilan als Reduktionsmittel beinhaltet.

24. Verfahren nach Anspruch 23, wobei die Lewis-Säure TMSOTf ist.

25. Verfahren nach Anspruch 24, wobei 0,2 Äquivalente von TMSOTf verwendet werden.

26. Verfahren nach einem der Ansprüche 23 bis 25, wobei die Reaktionstemperatur auf einen Wert im Bereich von -78°C bis-30°C geregelt wird.

27. Verfahren nach einem der Ansprüche 23 bis 26, wobei das Trioxanderivat eine Verbindung nach Anspruch 8 umfasst.

28. Verfahren nach einem der Ansprüche 23 bis 27, wobei das *O*-Trimethylsilyletherderivat von 10β-(2-Hydroxyethyl)-desoxoartemisinin hergestellt wird.

29. Verfahren zur Herstellung eines Trioxanderivats nach Anspruch 1, das die Behandlung von 10β-(2-Hydroxyethyl)-desoxoartemisinin mit Natriumhexamethyldisilazid und einem Dichlorphosphat beinhaltet.

30. Verfahren nach Anspruch 29, wobei zwei Äquivalente von 10β-(2-Hydroxyethyl)-desoxoartemisinin mit einem Äquivalent von Natriumhexamethyldisilazid und einem Äquivalent eines Dichlorphosphats in wasserfreiem THF behandelt werden.

31. Verfahren nach einem der Ansprüche 29 oder 30, wobei das Trioxanderivat eine Verbindung nach Anspruch 9 umfasst.

32. Verbindung nach einem der Ansprüche 1 bis 10 oder ein pharmazeutisch akzeptables Salz davon zur Verwendung als Medikament.

33. Verbindung nach Anspruch 32, wobei das Medikament ein Medikament zur Behandlung von Malaria oder Krebs ist.

34. Pharmazeutische Zusammensetzung, die eine Verbindung nach einem der Ansprüche 1 bis 10 oder ein pharmazeutisch akzeptables Salz davon als Wirkstoff enthält.

35. Verwendung einer Verbindung nach einem der Ansprüche 1 bis 10 oder eines pharmazeutisch akzeptablen Salzes davon zur Herstellung eines Medikaments zur Behandlung oder Prophylaxe von Malaria.

36. Verwendung einer Verbindung nach einem der Ansprüche 1 bis 10 oder eines pharmazeutisch akzeptablen Salzes davon zur Herstellung eines Medikaments zur Behandlung von Krebs.

37. Produkt, das eine erste Verbindung nach einem der Ansprüche 1 bis 10 oder ein pharmazeutisch akzeptables Salz davon, eine zweite eisenhaltige Verbindung als Kombipräparat zur simultanen, separaten oder aufeinander folgenden Verwendung zur Behandlung von Krebs enthält.

38. Produkt nach Anspruch 37, wobei die erste und die zweite Verbindung für eine aufeinander folgende Verwendung vorgesehen sind, wobei die zweite eisenhaltige Verbindung zuerst verwendet wird.

39. Produkt nach Anspruch 37 oder 38, wobei die zweite eisenhaltige Verbindung ein Eisensalz ist, das ausgewählt ist aus Eisen(II)-fumarat, Eisen(II)-sulfat, Eisen(II)-carbonat, Eisen(II)-citrat, Eisen(II)-gluconat, Eisen(II)-lactat und Eisen(II)-maleat.

40. Produkt nach Anspruch 37 oder 38, wobei die zweite eisenhaltige Verbindung ein Eisenkomplex ist, der ausgewählt ist aus Ferrocholinat, Ferroglycinsulfat, Dextraneisenkomplex, peptonisiertem Eisen, Eisen-Sorbitex, mit Zucker angereichertem Eisen, mit einem Eisenbindeprotein komplexiertem Eisen und mit einem Glykoprotein komplexiertem Eisen.

41. Produkt nach einem der Ansprüche 37 bis 40, wobei das Produkt ferner ein oder mehrere andere Agenzien umfasst, die zur Behandlung von Tumoren bekanntlich von Nutzen sind.

42. Produkt nach Anspruch 41, wobei das/die eine oder mehreren Agens/Agenzien ausgewählt ist/sind aus Androgeninhibitoren, Antiöstrogenen, Antimetaboliten und zytotoxischen Agenzien.

## Revendications

1. Composé ayant la formule générale suivante: 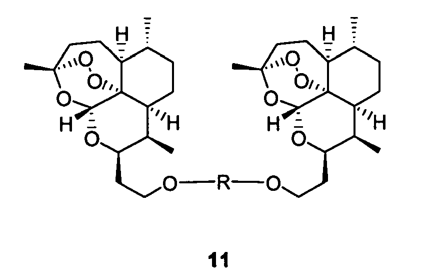 ou un sel de ce composé qui est pharmaceutiquement acceptable,
dans lequel R est sélectionné parmi le groupement comprenant les produits suivants substitués optionnellement: alkyle, aryle, ester de bisalkyle ou de bisaryle, éther de bisalkyle ou de bisaryle, acétal, cétal, boronate, éther de silyle, carbamate, carbonate, sulfate, sulfonate, phosphate et phosphonate.

2. Composé selon la revendication 1, **caractérisé en ce que** R comporte un groupement d'alkyles ou d'aryles substitué optionnellement et comprenant 1-20 atomes de carbone.

3. Composé selon la revendication 2, **caractérisé en ce que** R comporte un groupement d'alkyles ou d'aryles substitué optionnellement et comprenant 1-12 atomes de carbone.

4. Composé selon l'une quelconque des revendications 1 to 3, **caractérisé en ce que** R comporte un groupement d'alkyles ou d'aryles ayant un substituant sélectionné parmi des amines primaires, secondaires et tertiaires; des groupements contenant des halogènes; des alcools et des dérivés d'alcools; et des acides carboxyliques et des dérivés de ces acides.

5. Composé selon la revendication 4, **caractérisé en ce que** R est un groupement d'alkyles ou d'aryles ayant un substituant sélectionné parmi des amines primaires, secondaires et tertiaires.

6. Composé selon la revendication 1, **caractérisé en ce que** R est un phosphate.

7. Composé selon la revendication 1, **caractérisé en ce que** R est sélectionné parmi les groupements suivants:

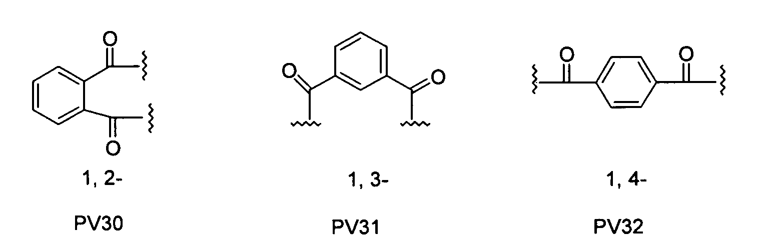

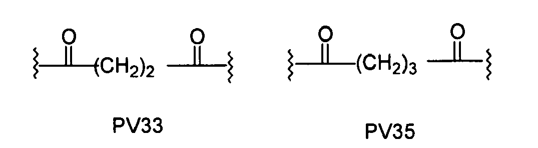

8. Composé selon la revendication 1, **caractérisé en ce que** R est sélectionné parmi les groupements suivants:

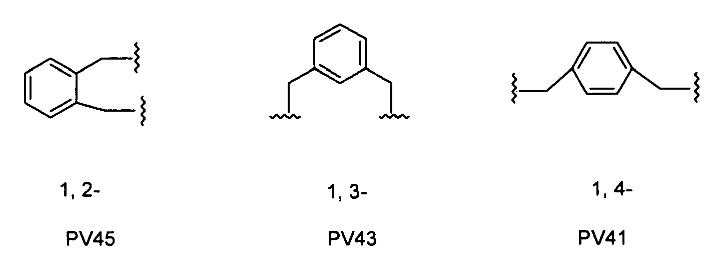

9. Composé selon la revendication 1, **caractérisé en ce que** R est sélectionné parmi les groupements suivants:

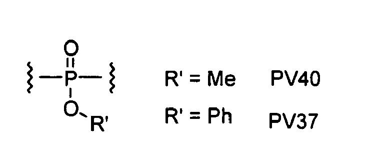

10. Composé selon la revendication 1, **caractérisé en ce que** R est sélectionné parmi les groupements suivants:

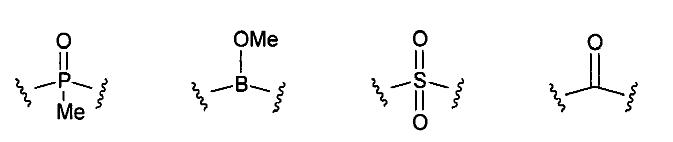

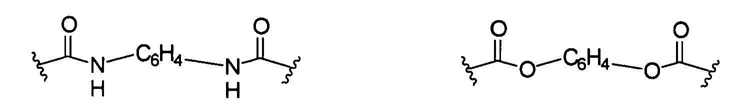

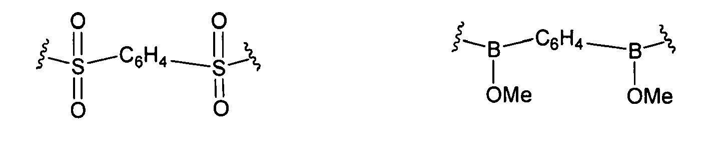

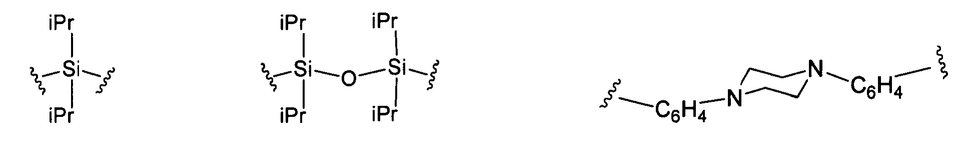

11. Procédé servant à produire un composé selon la revendication 1, **caractérisé en ce que** le procédé comporte la réaction du C-10 benzoate dihydroartemesinin avec de l'allyltriméthylsilane en présence d'un acide de Lewis faible pour former du 10β-allyldeoxoartemesinin, et aussi l'ozonolyse du 10β-allyldeoxoartemesinin et la réduction ultérieure de l'ozonide pour former du 10β-(2-hydroxyéthyl) deoxoartemesinin.

12. Procédé selon la revendication 11, **caractérisé en ce que** l'acide de Lewis faible est le chlorure de zinc.

13. Procédé selon l'une des revendications 11 ou 12, **caractérisé en ce que** le C-10-α-benzoate cristallin est mis en réaction avec de l'allyltriméthylsilane en présence de chlorure de zinc dans du dichloroéthane anhydre faisant fonction de solvant.

14. Procédé selon l'une quelconque des revendications 11 à 13, **caractérisé en ce que** la réaction se déroule en présence d'un agent de déshydratation.

15. Procédé selon la revendication 14, **caractérisé en ce que** l'agent de déshydratation comporte des cribles moléculaires de 4Å.

16. Procédé selon l'une quelconque des revendications 11 à 15, **caractérisé en ce que** la réaction se déroule sous atmosphère inerte.

17. Procédé selon l'une quelconque des revendications 11 à 16, **caractérisé en ce que** la réaction se déroule à une température comprise entre -10°C et 0°C.

18. Procédé selon l'une quelconque des revendications 11 à 17, **caractérisé en ce que** la durée de la réaction est de 4 heures.

19. Procédé selon l'une quelconque des revendications 11 à 18, **caractérisé en ce que** l'ozonide est réduit *in situ*.

20. Procédé de préparation d'un dérivé de trioxane selon la revendication 1, qui consiste entre autres à traiter le 10β-(2-hydroxyethyl)-deoxoartemisinin à l'aide d'un chlorure d'acide et d'une quantité catalytique de 4-(diméthylamino)pyridine.

21. Procédé selon la revendication 20, **caractérisé en ce que** deux équivalents de 10β-(2-hydroxyethyl)-deoxoartemisinin sont traités à l'aide d'un équivalent de chlorure d'acide.

22. Procédé selon l'une des revendications 20 ou 21, **caractérisé en ce que** le dérivé de trioxane comprend un composé selon la revendication 7.

23. Procédé de préparation d'un dérivé de trioxane selon la revendication 1, comportant l'ethérification bis-réductrice douce du dérivé d'éther *O*-trimethylsilyl suivant: 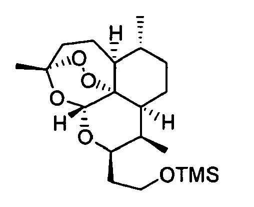 utilisant un bis-aldéhyde en présence d'un acide de Lewis et de triéthylsilane faisant fonction d'agent réducteur.

24. Procédé selon la revendication 23, **caractérisé en ce que** l'acide de Lewis est le TMSOTf.

25. Procédé selon la revendication 24, **caractérisé en ce que** 0,2 équivalents de TMSOTf sont utilisés.

26. Procédé selon l'une quelconque des revendications 23 à 25, **caractérisé en ce que** la température de réaction est contrôlée entre -78°C et -30°C.

27. Procédé selon l'une quelconque des revendications 23 à 26, **caractérisé en ce que** le dérivé de trioxane comprend un composé selon la revendication 8.

28. Procédé selon l'une quelconque des revendications 23 à 27, **caractérisé en ce que** le dérivé d'éther *O*-trimethylsilyl est préparé à partir de 10β-(2-hydroxyethyl)-deoxoartemisinin.

29. Procédé de préparation d'un dérivé de trioxane selon la revendication 1, comportant le traitement de 10β-(2-hydroxyethyl)-deoxoartemisinin à l'aide d'hexaméthyldisilazide de sodium et d'un dichlorophosphate.

30. Procédé selon la revendication 29, **caractérisé en ce que** deux équivalents de 10β-(2-hydroxyethyl)-deoxoartemisinin sont traités à l'aide d'un équivalent d'hexamethyldisilazide de sodium et d'un équivalent de dichlorophosphate, dans du THF anhydre.

31. Procédé selon l'une des revendications 29 ou 30, **caractérisé en ce que** le dérivé de trioxane comprend un composé selon la revendication 9.

32. Composé selon l'une quelconque des revendications 1 à 10, ou un sel de ce composé pharmaceutiquement acceptable, qui sera utilisé comme médicament.

33. Composé selon la revendication 32, **caractérisé en ce que** le médicament sert pour le traitement du paludisme ou du cancer.

34. Composition pharmaceutique contenant un composé selon l'une quelconque des revendications 1 à 10, ou un sel de ce composé pharmaceutiquement acceptable, faisant fonction d'ingrédient actif.

35. Utilisation d'un composé selon l'une quelconque des revendications 1 à 10, ou un sel de ce composé pharmaceutiquement acceptable, dans la fabrication d'un médicament servant pour le traitement ou la prophylaxie du paludisme.

36. Utilisation d'un composé selon l'une quelconque des revendications 1 à 10, ou un sel de ce composé pharmaceutiquement acceptable, dans la fabrication d'un médicament servant pour le traitement du cancer.

37. Produit contenant un premier composé selon l'une quelconque des revendications 1 to 10, ou un sel de ce composé pharmaceutiquement acceptable, un deuxième composé contenant du fer, servant de préparation combinée à usage simultané, séparé ou séquentiel dans le traitement des cancers.

38. Produit selon la revendication 37, **caractérisé en ce que** les premier et deuxième composés sont à usage séquentiel, le deuxième composé qui contient du fer étant utilisé en premier.

39. Produit selon l'une des revendications 37 ou 38, **caractérisé en ce que** le deuxième composé contenant du fer est un sel de fer sélectionné parmi le fumarate ferreux, le sulfate ferreux, le carbonate ferreux, le citrate ferreux, le gluconate ferreux, le lactate ferreux et le maléate ferreux.

40. Produit selon l'une des revendications 37 ou 38, **caractérisé en ce que** le deuxième composé contenant du fer est un complexe de fer sélectionné parmi le ferrocholinate, le sulfate de ferroglycine, le complexe fer dextran, le fer peptonisé, le sorbitex de fer, le fer sacchariné, le fer complexé avec une protéine servant à lier le fer, et le fer complexé avec une glycoprotéine.

41. Produit selon l'une quelconque des revendications 37 à 40, **caractérisé en ce que** le produit comprend par ailleurs un ou plusieurs autres agents connus pour leur utilité dans le traitement des tumeurs.

42. Produit selon la revendication 41, **caractérisé en ce que** l'autre ou les autres agents sont sélectionnés parmi les inhibiteurs d'androgènes, les anti-oestrogènes, les anti-métabolites et les agents cytotoxiques.
